# EUROPEAN PATENT APPLICATION

(11) **EP 4 552 642 A1**
(43) Date of publication of application: **14.05.2025**
(21) Application number: 23835561.4
(22) Date of filing: 05.07.2023
(51) Int. Cl.: A61K 31/423, A61P 21/06, A61P 43/00

(54) **AGENT FOR REDUCING BLOOD MYOSTATIN LEVEL**

(30) Priority: 06.07.2022 JP 2022109010
(71) Applicant: KOWA COMPANY, LTD., Naka-ku Nagoya-shi Aichi 460-8625 (JP)
(72) Inventor: TANIGAWA, Ryohei, Tokyo 103-8433 (JP)
(74) Representative: Plougmann Vingtoft a/s
(86) International application number: PCT/JP2023/024914
(87) International publication number: WO 2024/010030

(57) **Abstract**

A novel preventive and/or therapeutic agent useful in the prevention and/or treatment of a disease whose symptom is alleviated or improved by the inhibition of myostatin. The present invention relates to an agent for preventing and/or treating a disease whose symptom is alleviated or improved by the inhibition of myostatin, the agent including a therapeutically effective dose of (R)-2-[3-[[N-(benzoxazol-2-yl)-N-3-(4-methoxyphenoxy)propyl]aminomethyl]phenoxy]butyric acid, a salt thereof, or a solvate thereof.

## Description

### Field of the Invention

The present invention relates to an agent for reducing a blood myostatin level.

### Background of the Invention

Myostatin (another name: growth differentiation factor-8 (GDF8)) is a secretory protein produced from a skeletal muscle cell and suppresses the growth of a skeletal muscle, and the protein belongs to a TGF-β superfamily. It has been known from the phenotype of myostatin gene abnormality in an animal and a human, and observation with an animal model that the inhibition of myostatin shows muscle hypertrophy.

Attention has been paid to the function of myostatin, and the application of its inhibition to the treatment of an amyotrophic disease such as muscular dystrophy has been expected. An attempt has heretofore been made to develop a drug that exhibits muscle hypertrophy in an animal model, such as a neutralizing antibody for myostatin, an antagonist for myostatin such as a soluble activin IIB receptor, or an analog of follistatin serving as a physiological antagonist in the TGF-β superfamily. However, no drug has been known that was put into practical use as a therapeutic drug for a human.

As other approaches, attention has been paid to the fact that fenofibrate known as a PPARα agonist suppresses the expression of myostatin (Non Patent Document 1), and an investigation on its effect on a Duchenne muscular dystrophy model animal has been reported (Non Patent Document 2). However, each of those investigations is an investigation in a rodent animal model, and hence a clinical effect of fenofibrate in a human is uncertain.

Meanwhile, Patent Document 1 discloses that a compound represented by the following formula (1), a salt thereof, or a solvate thereof has a selective PPARα-activating effect, and is hence useful as a preventive and/or therapeutic drug for hyperlipidemia, arteriosclerosis, diabetes, a diabetic complication (e.g., diabetic nephropathy), inflammation, a heart disease, or the like that is not accompanied by a weight gain or obesity in a mammal including a human: 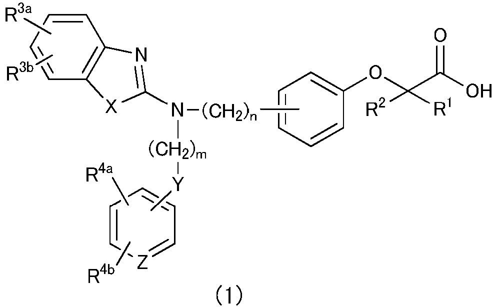 where R¹ and R² may be identical to or different from each other, and each represents a hydrogen atom, a methyl group, or an ethyl group, R^{3a}, R^{3b}, R^{4a}, and R^{4b} may be identical to or different from each other, and each represents a hydrogen atom, a halogen atom, a nitro group, a hydroxy group, a C₁₋₄ alkyl group, a trifluoromethyl group, a C₁₋₄ alkoxy group, a C₁₋₄ alkylcarbonyloxy group, a di-C₁₋₄ alkylamino group, a C₁₋₄ alkylsulfonyloxy group, a C₁₋₄ alkylsulfonyl group, a C₁₋₄ alkylsulfinyl group, or a C₁₋₄ alkylthio group, or R^{3a} and R^{3b}, or R^{4a} and R^{4b} are bonded to each other to represent an alkylenedioxy group, X represents an oxygen atom, a sulfur atom, or N-R⁵ (R⁵ represents a hydrogen atom, a C₁₋₄ alkyl group, a C₁₋₄ alkylsulfonyl group, or a C₁₋₄ alkyloxycarbonyl group), Y represents an oxygen atom, a S(O)ₗ group ("l" represents a number of from 0 to 2), a carbonyl group, a carbonylamino group, an aminocarbonyl group, a sulfonylamino group, an aminosulfonyl group, or an NH group, Z represents CH or N, "n" represents a number of from 1 to 6, and "m" represents a number of from 2 to 6.

However, there is no description or suggestion of what effect such a compound has on myostatin.

### Prior Art Documents

### Patent Document

[Patent Document 1] WO 2005/023777 A1

### Non Patent Documents

[Non Patent Document 1] Am J Physiol-Endoclinol Metab. (2011) 300, E790-E799
[Non Patent Document 2] Br J Pharmacol. (2021) 179, 1237-1250

### Summary of the Invention

An objective of the present invention is to provide a novel preventive and/or therapeutic agent useful in the prevention and/or treatment of a disease on which a therapeutic effect can be expected by inhibition of myostatin.

To achieve the above objective, the present inventors made energetic investigations, and as a result, found that to their complete surprise, a compound disclosed as Example 85 in Patent Document 1 described above, that is, (R)-2-[3-[[N-(benzoxazol-2-yl)-N-3-(4-methoxyphenoxy)propyl]aminomethyl]phenoxy]butyric acid (hereinafter may be referred to as "compound A" or "pemafibrate") reduced a blood myostatin concentration, and was hence useful in the prevention and/or treatment of a disease on which a therapeutic effect was expected by the inhibition of myostatin. In this way, the inventors completed the present invention.

That is, the present invention provides the following [1] to [28].
[1] An agent for reducing a blood myostatin level, comprising, as an active ingredient, (R)-2-[3-[[N-(benzoxazol-2-yl)-N-3-(4-methoxyphenoxy)propyl]aminomethyl]phenoxy]butyric acid, a salt thereof, or a solvate thereof.
[2] An inhibitor of myostatin, comprising (R)-2-[3-[[N-(benzoxazol-2-yl)-N-3-(4-methoxyphenoxy)propyl]aminomethyl]phenoxy]butyric acid, a salt thereof, or a solvate thereof, as an active ingredient.
[3] An agent for preventing and/or treating an amyotrophic disease, comprising (R)-2-[3-[[N-(benzoxazol-2-yl)-N-3-(4-methoxyphenoxy)propyl]aminomethyl]phenoxy]butyric acid, a salt thereof, or a solvate thereof, as an active ingredient.
[4] The agent according to [3], wherein the amyotrophic disease is Duchenne muscular dystrophy.
[5] The agent according to [3], wherein the amyotrophic disease is sarcopenia.
[6] A pharmaceutical composition for preventing and/or treating an amyotrophic disease, comprising a therapeutically effective dose of (R)-2-[3-[[N-(benzoxazol-2-yl)-N-3-(4-methoxyphenoxy)propyl]aminomethyl]phenoxy]butyric acid, a salt thereof, or a solvate thereof.
[7] The pharmaceutical composition according to [6], wherein the amyotrophic disease is Duchenne muscular dystrophy.
[8] The pharmaceutical composition according to [6], wherein the amyotrophic disease is sarcopenia.
[9] A method of reducing a blood myostatin level, comprising administering an effective dose of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.
[10] A method of inhibiting myostatin, comprising administering an effective dose of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.
[11] A method of preventing and/or treating an amyotrophic disease, comprising administering an effective dose of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.
[12] The method according to [11], wherein the amyotrophic disease is Duchenne muscular dystrophy.
[13] The method according to [11], wherein the amyotrophic disease is sarcopenia.
[14] Use of (R)-2-[3-[[N-(benzoxazol-2-yl)-N-3-(4-methoxyphenoxy)propyl]aminomethyl]phenoxy]butyric acid, a salt thereof, or a solvate thereof for reducing a blood myostatin level.
[15] Use of (R)-2-[3-[[N-(benzoxazol-2-yl)-N-3-(4-methoxyphenoxy)propyl]aminomethyl]phenoxy]butyric acid, a salt thereof, or a solvate thereof for inhibiting myostatin.
[16] Use of (R)-2-[3-[[N-(benzoxazol-2-yl)-N-3-(4-methoxyphenoxy)propyl]aminomethyl]phenoxy]butyric acid, a salt thereof, or a solvate thereof for preventing and/or treating an amyotrophic disease.
[17] The use according to [16], wherein the amyotrophic disease is Duchenne muscular dystrophy.
[18] The use according to [16], wherein the amyotrophic disease is sarcopenia.
[19] Use of (R)-2-[3-[[N-(benzoxazol-2-yl)-N-3-(4-methoxyphenoxy)propyl]aminomethyl]phenoxy]butyric acid, a salt thereof, or a solvate thereof for production of an agent for reducing a blood myostatin level.
[20] Use of (R)-2-[3-[[N-(benzoxazol-2-yl)-N-3-(4-methoxyphenoxy)propyl]aminomethyl]phenoxy]butyric acid, a salt thereof, or a solvate thereof for production of a myostatin inhibitor.
[21] Use of (R)-2-[3-[[N-(benzoxazol-2-yl)-N-3-(4-methoxyphenoxy)propyl]aminomethyl]phenoxy]butyric acid, a salt thereof, or a solvate thereof for production of a pharmaceutical composition for preventing and/or treating an amyotrophic disease.
[22] The use according to [21], wherein the amyotrophic disease is Duchenne muscular dystrophy.
[23] The use according to [21], wherein the amyotrophic disease is sarcopenia.
[24] (R)-2-[3-[[N-(Benzoxazol-2-yl)-N-3-(4-methoxyphenoxy)propyl]aminomethyl]phenoxy]butyric acid, a salt thereof, or a solvate thereof for use in reduction of a blood myostatin level.
[25] (R)-2-[3-[[N-(Benzoxazol-2-yl)-N-3-(4-methoxyphenoxy)propyl]aminomethyl]phenoxy]butyric acid, a salt thereof, or a solvate thereof for use in inhibition of myostatin.
[26] (R)-2-[3-[[N-(Benzoxazol-2-yl)-N-3-(4-methoxyphenoxy)propyl]aminomethyl]phenoxy]butyric acid, a salt thereof, or a solvate thereof for use in prevention and/or treatment of an amyotrophic disease.
[27] The (R)-2-[3-[[N-(benzoxazol-2-yl)-N-3-(4-methoxyphenoxy)propyl]aminomethyl]phenoxy]butyric acid, a salt thereof, or a solvate thereof according to [26], wherein the amyotrophic disease is Duchenne muscular dystrophy.
[28] The (R)-2-[3-[[N-(benzoxazol-2-yl)-N-3-(4-methoxyphenoxy)propyl]aminomethyl]phenoxy]butyric acid, a salt thereof, or a solvate thereof according to [26], wherein the amyotrophic disease is sarcopenia.

### Advantageous Effects of Invention

The present invention provides a novel agent useful in the prevention and/or treatment of a disease on which a therapeutic effect can be expected by the inhibition of myostatin. The present invention provides a novel preventive and/or therapeutic alternative to a patient of the disease on which the therapeutic effect can be expected by inhibition of myostatin.

### Brief Description of Drawing

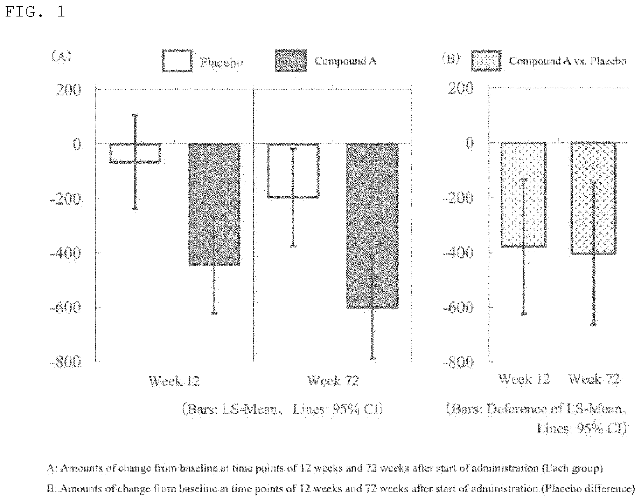

FIG. 1 shows the amounts of a change in fasting blood myostatin concentration (pg/mL) at the time of the administration of the compound A (0.4 mg per day) to a patient of a nonalcoholic fatty liver disease.

### Embodiments for Carrying out the Invention

(R)-2-[3-[[N-(Benzoxazol-2-yl)-N-3-(4-methoxyphenoxy)propyl]aminomethyl]phenoxy]butyric acid (compound A) to be used in the present invention is represented by the following chemical formula (A):

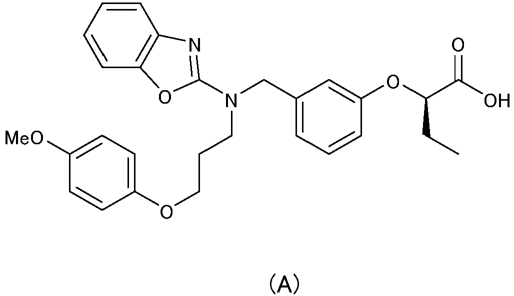

The compound A may be produced in accordance with, for example, a method described in WO 2005/023777 A1. The compound may be formulated to a preparation in accordance with a method described in a literature. A preparation containing the compound A is approved as a therapeutic agent for hyperlipidemia "PARMODIA (registered trademark) TABLET" in Japan, and the "PARMODIA TABLET" may be used.

In one embodiment of the present invention, a salt or a solvate of the compound A may be used. The salt and the solvate may each be produced by an ordinary method. While the salt of the compound A is not particularly limited as long as the salt is pharmaceutically acceptable, examples thereof include: alkali metal salts, such as a sodium salt and a potassium salt; alkaline earth metal salts, such as a calcium salt and a magnesium salt; organic base salts, such as an ammonium salt and a trialkylamine salt; mineral acid salts, such as a hydrochloride and a sulfate; and organic acid salts such as an acetate. Examples of the solvate of the compound A or of the salt thereof may include a hydrate and an alcohol adduct (e.g., an ethanol adduct).

As described in Examples described hereafter, the compound A reduces a blood myostatin concentration. It is conceivable from the fact that the administration of the compound A reduces the blood myostatin concentration that the compound A has a suppressing effect on the synthesis of myostatin in a body or a promoting effect on the discharge of myostatin. Accordingly, the compound A, a salt thereof, or a solvate thereof may be an active ingredient of an agent for reducing a blood myostatin level. The compound A, a salt thereof, or a solvate thereof is useful in the prevention and/or treatment of a disease whose symptom is alleviated or improved by the inhibition of myostatin through its reduction in blood myostatin concentration.

Unless otherwise stated, the term "agent for reducing a blood myostatin level" as used herein refers to, for example, an agent whose application reduces a blood myostatin level to alleviate or improve the symptom of, for example, a disease whose symptom is alleviated or improved by the inhibition of myostatin.

Unless otherwise stated, the term "myostatin inhibitor" as used herein refers to, for example, an agent that inhibits the function of myostatin in a living organism to alleviate or improve the symptom of, for example, a disease whose symptom is alleviated or improved by the inhibition of myostatin. The mode of inhibition of myostatin function is not particularly limited, and examples thereof include: the suppression of the expression of myostatin or the inhibition of the expression; the inhibition of the binding thereof to a myostatin receptor; the competitive inhibition of the binding to the myostatin receptor; and the decomposition of myostatin.

The term "disease whose symptom is alleviated or improved by the inhibition of myostatin" as used herein refers to, for example, an amyotrophic disease or a cancer cachexia. Reduction in body fat can be expected from the inhibition of myostatin.

Examples of the amyotrophic disease may include: a disease resulting from myogenic amyotrophy such as muscular dystrophy; a disease resulting from neurogenic amyotrophy, such as amyotrophic lateral sclerosis (ALS), spinal muscular atrophy (SMA), or spinal and bulbar muscular atrophy; and sarcopenia. Of those, muscular dystrophy or sarcopenia is preferred in the present invention.

The muscular dystrophy is a generic term for hereditary muscle diseases in each of which amyotrophy and muscle weakness gradually progress while the destruction, degeneration (myonecrosis), and regeneration of muscle fibers are repeated, and the dystrophy shows a so-called dystrophy change in which the loss of the structure of a muscle fiber bundle, which is characterized by variation in size of the muscle fibers, the formation thereof into circles, an increase in central nuclei thereof, the hyperplasia of the connective tissues thereof, and the fatty metamorphosis thereof, occurs.

Examples of the muscular dystrophy may include: chromosomal recessive hereditary muscular dystrophy, such as Duchenne muscular dystrophy or Becker muscular dystrophy; congenital muscular dystrophy, such as Fukuyama muscular dystrophy, Ullrich muscular dystrophy, merosin deficiency, integrin deficiency, or Walker-Warburg syndrome; limb-girdle muscular dystrophy; Miyoshi muscular dystrophy; facioscapulohumeral muscular dystrophy; and myotonic dystrophy. Of those, a chromosomal recessive hereditary muscular dystrophy, such as Duchenne muscular dystrophy or Becker muscular dystrophy, is preferred in the present invention, and Duchenne muscular dystrophy is more preferred.

In one aspect of the present invention, the compound A, a salt thereof, or a solvate thereof, when used as a drug, may be formulated into a preparation, such as a tablet, a capsule, a granule, a powder, a lotion, an ointment, an injection, or a suppository, through use of any other pharmaceutically acceptable carrier as required. Those preparations may each be produced by a known method. Examples of the pharmaceutically acceptable carrier may include an excipient, a disintegrator, a binder, a lubricant, a plasticizer, a glidant, a diluent, a solubilizer, a suspending agent, an isotonizing agent, a pH adjuster, a buffer, a stabilizer, a coating agent, a colorant, a taste-masking agent, and an odor-masking agent.

In one aspect of the present invention, the compound A, a salt thereof, or a solvate thereof may be administered orally or parenterally, and oral administration is preferred. The therapeutically effective dose of the compound A, the salt thereof, or the solvate thereof, and the number of times of the administration thereof may be appropriately set by a person skilled in the art, though the dose and the number vary depending on, for example, the body weight, age, sex, and symptom of a patient. For example, for a normal adult, the compound A may be administered in a dose of from 0.05 mg to 0.8 mg per day in one to three portions, and is administered preferably in a dose of from 0.1 mg to 0.4 mg per day in one or two portions, more preferably in a dose of from 0.2 mg to 0.4 mg per day in 1 or 2 portions.

### Examples

The present invention is more specifically described below by way of Examples. However, these Examples are not intended to limit the present invention.

### Example 1: Investigation on Effect of Compound A on Blood Myostatin

An effect on blood myostatin at the time of the oral administration of 0.4 mg per day of the compound A (a 0.2-mg tablet of the compound A was administered twice a day at the time of fasting) to a patient of a nonalcoholic fatty liver disease (NAFLD) for 72 weeks was investigated by comparison to the administration of a placebo.

FIG. 1 shows the amounts of a change (pg/mL) from the baselines of blood myostatin concentrations at the time points of 12 weeks and 72 weeks after the start of the administration. The numerical values of the baselines are shown in Table 1.

**Table 1**

| Baseline (pg/mL) | | |
|---|---|---|
| | Placebo | Compound A |
| n | 59 | 57 |
| Mean | 3708.08 | 3812.41 |
| SD | 1149.62 | 1362.47 |

It was clear from FIG. 1 that the administration of the compound A reduced the fasting blood myostatin concentration at 12 weeks and 72 weeks from the administration in a stepwise manner as compared to the placebo administration. Accordingly, it was found that the compound A is useful as a preventive and/or therapeutic agent for a disease whose symptom was alleviated or improved by inhibition of myostatin.

### Industrial Applicability

The present invention was completed on the basis that it was found for the first time that the compound A has a reducing effect on a fasting blood myostatin concentration. The present invention is useful as a drug for preventing and/or treating a disease whose symptom is alleviated or improved by inhibition of myostatin.

## Claims

1. An agent for reducing a blood myostatin level, comprising, as an active ingredient, (R)-2-[3-[[N-(benzoxazol-2-yl)-N-3-(4-methoxyphenoxy)propyl]aminomethyl]phenoxy]butyric acid, a salt thereof, or a solvate thereof.

2. An inhibitor of myostatin, comprising (R)-2-[3-[[N-(benzoxazol-2-yl)-N-3-(4-methoxyphenoxy)propyl]aminomethyl]phenoxy]butyric acid, a salt thereof, or a solvate thereof, as an active ingredient.

3. An agent for prevention and/or treatment of an amyotrophic disease, comprising (R)-2-[3-[[N-(benzoxazol-2-yl)-N-3-(4-methoxyphenoxy)propyl]aminomethyl]phenoxy]butyric acid, a salt thereof, or a solvate thereof, as an active ingredient,.

4. The agent according to claim 3, wherein the amyotrophic disease is Duchenne muscular dystrophy.

5. The agent according to claim 3, wherein the amyotrophic disease is sarcopenia.

6. A method of reducing a blood myostatin level, comprising administering an effective dose of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.

7. A method of inhibiting myostatin, comprising administering an effective dose of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.

8. A method of preventing and/or treating an amyotrophic disease, comprising administering an effective dose of (R)-2-[3-[[N-(benzoxazol-2-yl)-N-3-(4-methoxyphenoxy)propyl]aminomethyl]phenoxy]butyric acid, a salt thereof, or a solvate thereof to a patient in need thereof.

9. The method according to claim 8, wherein the amyotrophic disease is Duchenne muscular dystrophy.

10. The method according to claim 8, wherein the amyotrophic disease is sarcopenia.

11. Use of (R)-2-[3-[[N-(benzoxazol-2-yl)-N-3-(4-methoxyphenoxy)propyl]aminomethyl]phenoxy]butyric acid, a salt thereof, or a solvate thereof for production of an agent for reducing a blood myostatin level.

12. Use of (R)-2-[3-[[N-(benzoxazol-2-yl)-N-3-(4-methoxyphenoxy)propyl]aminomethyl]phenoxy]butyric acid, a salt thereof, or a solvate thereof for production of an inhibitor of myostatin.

13. Use of (R)-2-[3-[[N-(benzoxazol-2-yl)-N-3-(4-methoxyphenoxy)propyl]aminomethyl]phenoxy]butyric acid, a salt thereof, or a solvate thereof for production of a pharmaceutical composition for preventing and/or treating an amyotrophic disease.

14. The use according to claim 13, wherein the amyotrophic disease is Duchenne muscular dystrophy.

15. The use according to claim 13, wherein the amyotrophic disease is sarcopenia.

16. (R)-2-[3-[[N-(benzoxazol-2-yl)-N-3-(4-methoxyphenoxy)propyl]aminomethyl]phenoxy]butyric acid, a salt thereof, or a solvate thereof for use in reduction of a blood myostatin level.

17. (R)-2-[3-[[N-(benzoxazol-2-yl)-N-3-(4-methoxyphenoxy)propyl]aminomethyl]phenoxy]butyric acid, a salt thereof, or a solvate thereof for use in inhibition of myostatin.

18. (R)-2-[3-[[N-(benzoxazol-2-yl)-N-3-(4-methoxyphenoxy)propyl]aminomethyl]phenoxy]butyric acid, a salt thereof, or a solvate thereof for use in prevention and/or treatment of an amyotrophic disease.

19. The (R)-2-[3-[[N-(benzoxazol-2-yl)-N-3-(4-methoxyphenoxy)propyl]aminomethyl]phenoxy]butyric acid, a salt thereof, or a solvate thereof according to claim 18, wherein the amyotrophic disease is Duchenne muscular dystrophy.

20. The (R)-2-[3-[[N-(benzoxazol-2-yl)-N-3-(4-methoxyphenoxy)propyl]aminomethyl]phenoxy]butyric acid, a salt thereof, or a solvate thereof according to claim 18, wherein the amyotrophic disease is sarcopenia.
